(19) **Europäisches Patentamt**
**European Patent Office**
**Office européen des brevets**

(11) **EP 2 435 850 B1**

(12) **EUROPEAN PATENT SPECIFICATION**

(45) Date of publication and mention
of the grant of the patent:
**07.08.2013 Bulletin 2013/32**

(51) Int Cl.:
***G01S 7/52*** *(2006.01)*

(21) Application number: **10723802.4**

(22) Date of filing: **30.04.2010**

(86) International application number:
**PCT/JP2010/057980**

(87) International publication number:
**WO 2010/137453 (02.12.2010 Gazette 2010/48)**

(54) **ULTRASOUNG IMAGING MEASUREMENT APPARATUS USING ADAPTIVE DATA REDUCTION**

ULTRASCHALLBILDGEBUNGS-MESSVORRICHTUNG, DIE ADAPTIVE DATENREDUKTION
VERWENDET

APPAREIL DE MESURE PAR IMAGERIE ULTRASONORE UTILISANT UNE RÉDUCTION DES
DONNÉES ADAPTATIVE

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **27.05.2009 JP 2009127829**
**24.03.2010 JP 2010068568**

(43) Date of publication of application:
**04.04.2012 Bulletin 2012/14**

(60) Divisional application:
**13173824.7**

(73) Proprietor: **Canon Kabushiki Kaisha
Tokyo 146-8501 (JP)**

(72) Inventor: **TATEYAMA, Jiro
Tokyo 146-8501 (JP)**

(74) Representative: **TBK
Bavariaring 4-6
80336 München (DE)**

(56) References cited:
**EP-A2- 1 041 396     US-A- 5 349 525
US-A- 5 740 805     US-A- 5 970 025
US-B1- 6 716 172**

• **SHAN TIE-JUN ET AL: "Adaptive Beamforming
for Coherehnt Signals and Interference" IEEE
TRANSACTIONS ON ACOUSTICS, SPEECH AND
SIGNAL PROCESSING, IEEE INC. NEW YORK,
USA, vol. ASSP-33, no. 3, 1 June 1985
(1985-06-01), pages 527-536, XP002585452 ISSN:
0096-3518**
• **SUHYUN PARK ET AL: "Adaptive beamforming
for photoacoustic imaging using linear array
transducer" ULTRASONICS SYMPOSIUM, 2008.
IUS 2008. IEEE, IEEE, PISCATAWAY, NJ, USA, 2
November 2008 (2008-11-02), pages 1088-1091,
XP031443268 ISBN: 978-1-4244-2428-3**
• **VIOLA F ET AL: "Adaptive signal processing in
medical ultrasound beamforming"
ULTRASONICS SYMPOSIUM, 2005 IEEE
ROTTERDAM, THE NETHERLANDS 18-21 SEPT.
2005, PISCATAWAY, NJ, USA,IEEE LNKD- DOI:
10.1109/ULTSYM.2005.1603264, vol. 4, 18
September 2005 (2005-09-18), pages 1980-1983,
XP010899185 ISBN: 978-0-7803-9382-0**

EP 2 435 850 B1

**Description**

TECHNICAL FIELD

The present invention relates to a measurement apparatus, which receives an ultrasound emitted from within an object and acquires a tomographic image or a three-dimensional image inside the object, and particularly to a measurement apparatus, which receives an ultrasound and performs adaptive signal processing on an acquired received signal.

BACKGROUND ART

An ultrasound probe including a plurality of ultrasound transducers (ultrasound transducing devices) having an ultrasound transmitting/receiving function is used in a measurement apparatus for use in medical diagnosis. When an ultrasound beam formed by combining a plurality of ultrasounds is emitted to an object from each device of the ultrasound probe including such a plurality of devices, the ultrasound beam is reflected from a region of different acoustic impedance, namely, a boundary between tissues inside the object. Then, an ultrasound echo generated in such a manner is received and an image is constructed based on the intensity of the ultrasound echo. Thereby, conditions inside the object can be reproduced on a screen. Alternatively, there is a method of imaging inside of an object through a photoacoustic effect using an elastic wave which is received in such a manner that, pulsed light is emitted to inside the object and light energy is absorbed to cause adiabatic expansion, which, as a result, produces an elastic wave (hereinafter referred to as a photoacoustic wave which is an ultrasound).

Meanwhile, adaptive signal processing has been developed in the field of radar and the like. The adaptive signal processing refers to processing of adaptively controlling process parameters according to a propagation environment, capturing a desired wave, and suppressing an interference wave (noise component). Examples of the adaptive signal processing includes a directionally constrained minimization of power (DCMP) method for minimizing a signal power in a state in which a sensitivity to a specific direction (a desired wave arrival direction) is fixed when a plurality of devices receive ultrasounds and convert the waves to received signals (analog signals). Such adaptive signal processing is effective in improving spatial resolution (particularly spatial resolution in a lateral direction).

Here, it has been known that the above DCMP method is effective when a noise component and a desired wave are not correlated to each other, but the DCMP method cannot be applied as is when a noise component and a desired wave are correlated to each other. Specifically, when a noise component correlated to a desired wave is received, a directional received pattern is formed which has a sensitivity in opposite phase also in the noise component direction other than the desired wave direction. This is because in order to minimize an output signal, an attempt is made to approximate the output signal to zero by adding the noise component to the desired wave in opposite phase.

Meanwhile, when imaging using an ultrasound transmitting/receiving or a photoacoustic effect is performed, unlike the radar technical field, the noise component is highly correlated to the desired wave. This is because when an ultrasound is used for imaging, major noise components are caused by transmission waves reflected from a direction other than the desired wave direction and thus, the noise component is highly correlated to the desired wave. In addition, when a photoacoustic effect is used for imaging, incident light spreads over a wide range by the scattering effect, and thus there is a high possibility that ultrasounds occurring in the wide range are highly correlated to each other.

Spatial smoothing is a technique for allowing the DCMP to work even on such highly correlated noise. According to the spatial smoothing, a plurality of partial matrices is extracted from a correlation matrix and the extracted partial matrices are averaged to obtain a partial correlation matrix, which is used to calculate an optimal weight. This can avoid having the sensitivity in the noise component direction and thus an ultrasound diagnostic apparatus can also have the same effect as the DCMP of improving a spatial resolution in a lateral direction. The spatial smoothing is defined in "IEEE Trans. Acoust., Speech, Signal Process., Vol. ASSP-33, No.3, pp. 527-536 (June 1985) XP002585452. In addition, U.S. Patent No. 6,798,380 discloses a prior art using a Capon method, one of the spatial smoothing techniques, in a measurement apparatus, indicating that the calculation of partial correlation matrices becomes complicated when Capon beamforming is used.

As described above, in order to remove a noise component correlated to a desired wave, a measurement apparatus performing adaptive signal processing needs to use spatial smoothing. Therefore, the measurement apparatus needs to have a signal processing section capable of processing partial correlation matrices at high speeds. When a partial correlation matrix is signal-processed, the amount of data increases in proportion to an individual parameter such as the bit width of a received signal, the number of devices, and the sampling time. There is a problem that the data transfer time to a calculation section and the calculation time by the calculation section performing adaptive signal processing such as partial correlation matrix processing are too slow to catch up with an image display rewrite time (refresh rate).

Document US 5 349 525 A discloses an ultrasonic imaging system for displaying color flow images including a receiver which demodulates ultrasonic echo signals received by a transducer array and dynamically focuses the baseband echo signals. A color flow processor includes a frequency domain adaptive wall filter which automatically adjusts to changes in Doppler-shifted frequency and bandwidth of the wall signal components in the focused baseband echo signals after the echo signals have undergone Fourier transformation into the frequency domain. The mean Doppler-shifted frequency of the resulting filtered baseband echo signals is used to indicate velocity of moving scatterers and to control color in the displayed image.

Document US 5 970 025 A discloses a method and system for an ultrasound beamformer with an integrated circuit. Both digital transmit and digital receive beam formation are processed on a single integrated circuit receiver is provided. The transmit beamformer includes transmit logic and a digital to analog converter. The receive beamformer includes receive logic and an analog to digital converter. In one preferred embodiment, the integrated circuit includes a generic gate array with a designed logic metal layer for implementing the transmit and receive logic. The converters allow analog inputs and outputs, reducing the pin count. Reduction in pin count may permit cost reductions as process geometries become smaller. By placing both logic functions on one IC, fewer ICs are required in each system and the volume for that particular IC may increase, reducing the cost.

DISCLOSURE OF THE INVENTION

In order to solve the above problems, the present invention has been made, and an object of the present invention is to provide a measurement apparatus capable of providing high-speed signal processing for adaptive signal processing.

According to an aspect of the present invention there is provided a measurement apparatus as defined in claim 1.

Further aspects and features of the present invention are set out in the dependent claims.

The measurement apparatus for imaging using spatial smoothing can perform signal processing at high speeds.

Other features and advantages of the present invention will be apparent from the following description taken in conjunction with the accompanying drawings, in which like reference characters designate the same or similar parts throughout the figures thereof.

BRIEF DESCRIPTION OF THE DRAWINGS

FIG. 1 is a block diagram illustrating a configuration of an ultrasound diagnostic apparatus according to a first embodiment and a second embodiment.

FIG. 2 is a block diagram illustrating a configuration of a conventional ultrasound diagnostic apparatus.

FIG. 3 is an explanatory drawing explaining an internal configuration of a receiving section and a phase matching calculation section.

FIG. 4 is a configuration view of a data reducing section according to the first embodiment.

FIGS. 5A and 5B each is a configuration view of a data reducing section according to the second embodiment.

FIGS. 6A, 6B, 6C, and 6D each are an explanatory drawing explaining data reduction processes to the second embodiment.

FIG. 7 is a block diagram illustrating a configuration of an ultrasound diagnostic apparatus according to a third embodiment.

FIGS. 8A, 8B, 8C, and 8D each are an explanatory drawing explaining image display method of the third embodiment.

BEST MODE FOR CARRYING OUT THE INVENTION

Preferred embodiments of the present invention will now be described in detail in accordance with the accompanying drawings.

Hereinafter, embodiments of the present invention will be described in detail by referring to the accompanying drawings.

(Conventional ultrasonud diagnostic apparatus)

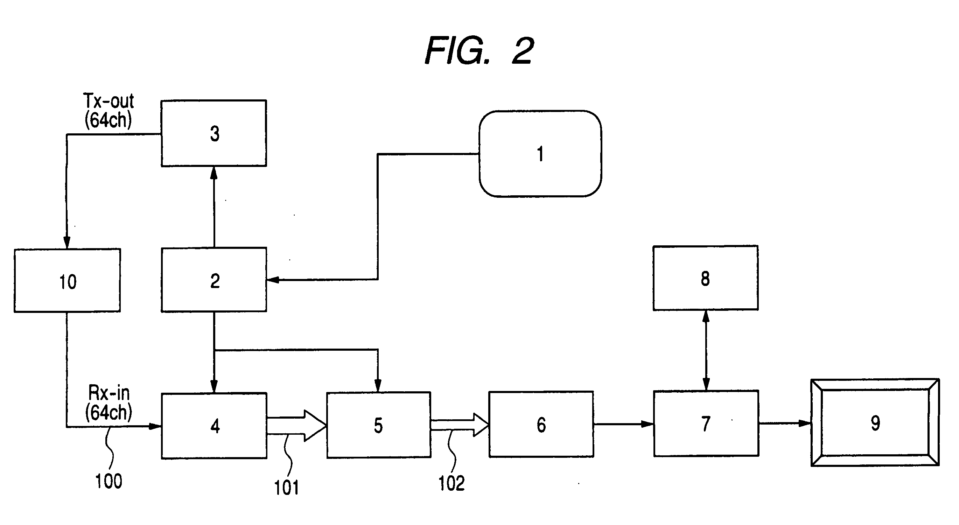

First, by referring to a block diagram of FIG. 2 illustrating a configuration of a conventional ultrasound diagnostic apparatus (measurement apparatus), an internal configuration of a general ultrasound diagnostic apparatus will be described. The internal configuration of the ultrasound diagnostic apparatus includes an ultrasound probe 10, an input operation section 1, a transmitting/receiving control section 2, a transmitting section 3, a receiving section 4 as a received signal processing unit, a phase matching calculation section 5, a signal processing section 6, a scan converter 7, an image data storage section 8, and an image display section 9.

The ultrasound probe 10 is used so as to be in contact with an object, and transmits and receives an ultrasound beam to and from the object. The ultrasound probe 10 includes a plurality of ultrasound transducers (ultrasound transducing devices), each of which transmits an ultrasound beam based on an applied drive signal, receives an ultrasound echo reflected and propagated in the object and converts the ultrasound echo to a received signal which is an analog signal, and outputs the received signal. The ultrasound transducers are arranged 1- or 2-dimensionally to constitute a transducer array (device array).

The ultrasound transducer includes oscillators, each having an electrode formed on both ends of a piezoelectric material (piezoelectric body) such as a piezoelectric ceramic exemplified by PZT (Pb (lead) Zirconate Titanate) and a polymer piezoelectric device exemplified by PVDF (PolyVinylidene DiFluoride). Alternatively, a plurality of kinds of devices having different conversion system may be used as the ultrasound transducer. For example, a configuration is made such that the aforementioned oscillator is used as a device transmitting an ultrasound; and an ultrasound transducer in an optical detection system is used as a device receiving the ultrasound. The ultrasound transducer in an optical detection system is to convert an ultrasound beam to a light signal for detection and, for example, includes Fabry-Perot resonators or fiber Bragg gratings. Alternatively, a capacitance ultrasound transducer may be used.

The input operation section 1 is used when an operator inputs an instruction and information to the ultrasound diagnostic apparatus. The input operation section 1 includes a keyboard, an adjustment knob, a pointing device including a mouse, and the like.

The transmitting/receiving control section 2 includes a processor and software. Based on the instruction and the information input from the input operation section 1, the transmitting/receiving control section 2 controls each block of the transmitting section 3, the receiving section 4, and the phase matching calculation section 5 of the ultrasound diagnostic apparatus.

The transmitting section 3 includes a plurality of channels of drive circuits, each generating a plurality of channels of drive signals (Tx-out) to be supplied to a plurality of ultrasound transducers. Here, as an example, the ultrasound diagnostic apparatus has a total of 64 channels. Here, one channel corresponds to one device.

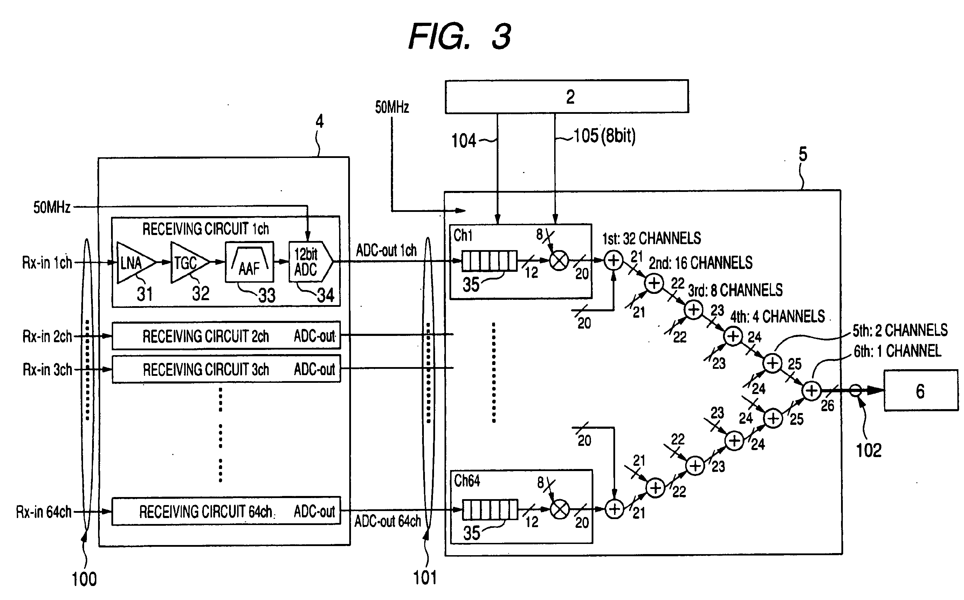

FIG. 3 illustrates an internal configuration of a receiving section 4 and a phase matching calculation section 5. The receiving section 4 receives analog signals from each ultrasound transducer. First, an LNA (Low Noise Amplifier) 31 of the receiving section 4 amplifies each analog received signal (Rx-in) 100. Then, a TGC (time gain compensation) amplifier 32 further amplifies the analog received signals. The analog amplification processing allows the received signal level to be matched with an input signal level of the A/D converter. The amplified analog signal output from the TGC amplifier 32 is input to an AAF (Anti Alias Filter) 33 in which LPF (Low Pass Filter) processing is performed for the purpose of removing aliasing noise. Further, the A/D converter 34 converts the analog signal to a digital signal and a 12-bit digital signal is generated at a sampling frequency of 50 MHz. The 12-bit digital signal is generated for each channel, and thus a total of 64 channels of digital signals are generated in FIG.3. Consequently, the transfer rate of the echo detection data 101, which is a digital signal, can be expressed in units of bps (bit per sec) which is a bit rate unit as follows.

$$\texttt{12 bits} \times \texttt{50 MHz} \times \texttt{64 chs} = \texttt{38.4 Gbps}$$

The phase matching calculation section 5 is a circuit performing delay and sum processing for matching the phase of the echo detection data 101, namely, received focus processing. The phase matching calculation section 5 applies a desired focus delay to a plurality of channels of echo detection data 101 stored in a FIFO (First In First Out) 35, and then performs sum processing. Thereby, phase-matched data 102 is generated which indicates ultrasound information along a desired scanline. Here, a shift register delay line, a digital micro delay device, a sum adder, and the like of the phase matching calculation section 5 includes hardware blocks using an FPGA and the like. Note that the phase matching calculation section 5 may include a CPU (central processing unit) and software, or a combination thereof.

The echo detection data 101 output from the A/D converter 34 and input to the phase matching calculation section 5 is stored in the FIFO 35 thereof for a specific period of time in order to obtain a focus delay adapted to delay amount data 104 supplied from the transmitting/receiving control section 2. Each channel of data undergoing focus delay is selected from the time series echo detection data 101 stored in the FIFO 35 and is multiplied by weight data 105 required for received focus processing. Each channel of data multiplied by the weight data 105 undergoes tournament (ladder) sum processing between adjacent channels. Finally, 64 channels of data are summed to output the phase-matched data 102 indicating ultrasound information along a desired scanline.

The 12-bit echo detection data 101 inputs to the phase matching calculation section 5 is multiplied by 8-bit weight data 105 to produce 20-bit data, which undergoes 64 channels of sum processing, and finally 26-bit data is output. The transfer rate of the phase-matched data 102 can be expressed in bit rate as follows.

```
26 bits × 50 MHz = 1.3 Gbps
```

As the input/output bit rate ratio, about 1/30 compression is achieved.

The signal processing section 6 performs an envelope detection and an STC (sensitivity time gain control) on the phase-matched data 102 undergoing received focus processing by the phase matching calculation section 5 to generate image data (image information) called an A mode. The A mode image data is 1-dimensional image data. While the data is temporarily stored in the image data storage section 8, the scan converter 7, which generates image data in units of frames, generates two-dimensional image (tomographic image) data called a B mode. The two-dimensional image data is output to the image display section 9 and is displayed as a tomographic image thereon. Note that three-dimensional image data can also be generated from two-dimensional image data by planarly operating the ultrasound probe so as to be displayed as a three-dimensional image.

(First embodiment)

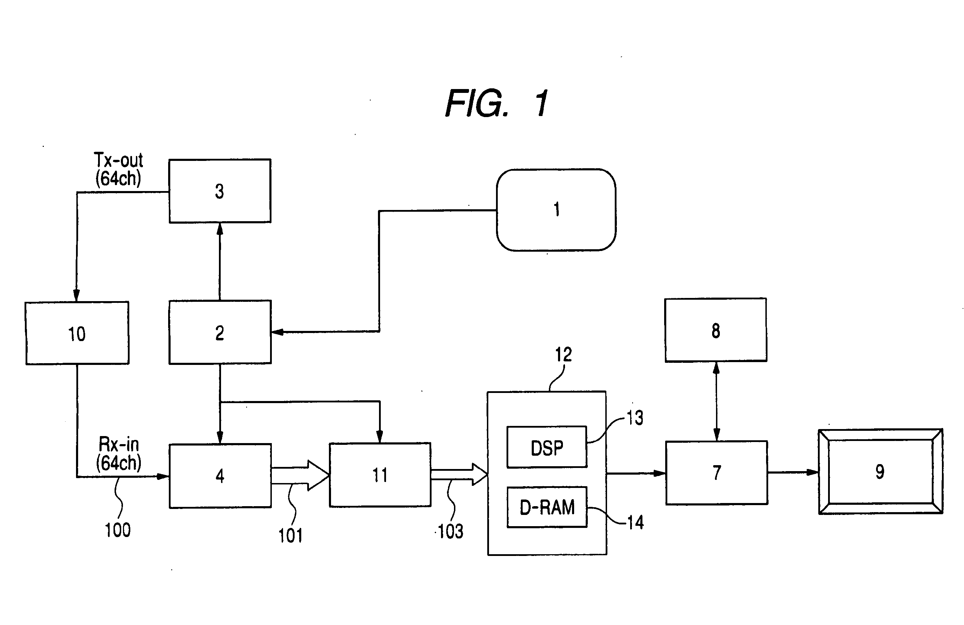

Next, FIG. 1 illustrates an ultrasound diagnostic apparatus according to a first embodiment of the present invention. FIG. 1 is different from FIG. 2 illustrating a conventional general ultrasound diagnostic apparatus in that the phase matching calculation section 5 and the signal processing section 6 are replaced with a data reducing section 11 as a data reducing unit and a calculation section 12 as a calculating unit respectively.

In order to improve the azimuth resolution, the present embodiment uses the DCMP method as adaptive signal processing. As described in "BACKGROUND ART", a received signal contains a noise component correlated to a desired wave, and thus spatial smoothing needs to be applied. According to the spatial smoothing, a plurality of partial matrices is extracted from a correlation matrix and the extracted partial matrices are averaged to obtain a partial correlation matrix, which is used to calculate an optimal weight. The partial correlation matrix $R_{pxx}$ can be calculated by the following expression. Note that N denotes the number of partial matrices to be extracted and M denotes the size of a partial matrix calculated by K-N+1. In addition, Zn denotes a weight coefficient when a partial matrix is averaged. Zn is a simple average for Zn = 1/N, but a Hamming window, a Hanning window, a Dolph-Chebycheff window, and the like can be used as the weight function.

(Expression 1)

$$R_{pxx} = \sum_{n=1}^{N} z_n E\left[ X_n(t) X_n^H(t) \right]$$

$$X_n(t) = \left[ X_n(t), X_{n+1}(t), \cdots, X_{n+M-1}(t) \right]^T \quad (n = 1, 2, \cdots, N)$$

A desired wave arrival direction is estimated from the thus calculated partial correlation matrix $R_{pxx}$, and based on the obtained information; an appropriate constraint condition is set to apply the DCMP method. Thereby, even when a noise component highly correlated to a desired wave is received, having the sensitivity in the noise component direction can be avoided. The calculation section 12 performs envelope detection, STC processing, etc. on the calculation result by the DCMP method to generate a mode image data (image information). The generated a mode image data is output to the scan converter 7.

The calculation section 12 is required to perform high-speed calculation on partial correlation matrices and high-speed calculation by the DCMP method to output image data. Therefore, the calculation section 12 may include a DSP (digital signal processor) 13 for performing high-speed signal processing. The calculation section 12 also includes a D-RAM 14 for reserving a storage memory area required for calculation and other hardware calculating units. Note that obviously, the DSP is not always required, and a general purpose processor may be used instead as long as the processor can operate at sufficiently high speeds.

When the calculation section 12 calculates partial correlation matrices using all channels of echo detection data 101, the time to generate one frame of image data is longer than the time for the conventional phase matching calculation section 5. When the refresh rate of a displayed image is delayed, the reproduced images are displayed like images viewed frame by frame. Moreover, in an equipment environment in which the costs and size of the measurement apparatus are limited, hardware reinforcement for increasing calculation speeds is also limited, and thus some simplification of calculation processing is required. In light of this, the ultrasound diagnostic apparatus of the present embodiment includes a new data reducing section 11 in order to reduce the amount of calculation data 103 to be input to the calculation section 12.

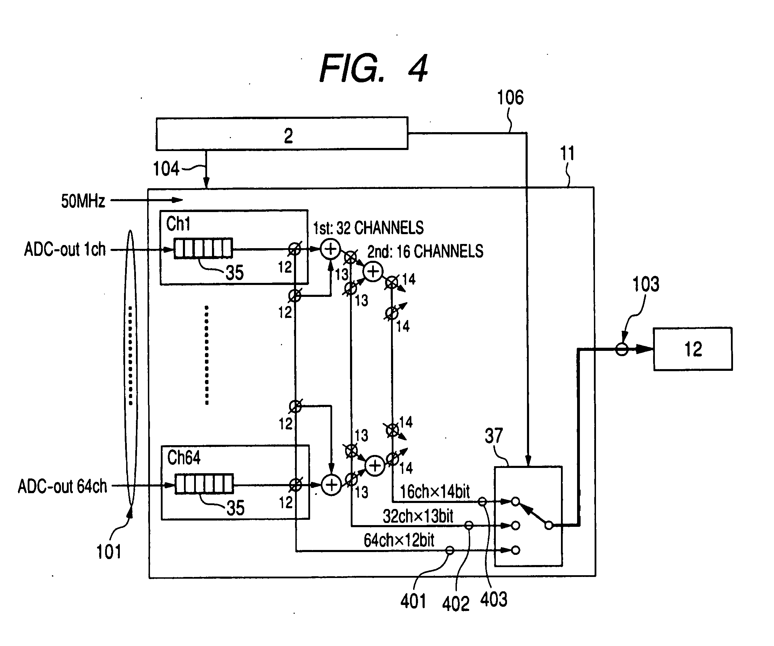

FIG. 4 illustrates a configuration example of the data reducing section 11 in which a part of the functions of the phase matching calculation section 5 is used. The data reducing section 11 of the present embodiment reduces the amount of data by adding the echo detection data between adjacent devices. In comparison with the phase matching calculation section 5, the data reducing section 11 needs no received focus processing, and thus does not need to have the weight data 105, and thus needs no weight multiplier connected to an FIFO stage output. With this, the number of bits of the adder for adjacent channels is reduced. For example, two channels of data are added to produce 13-bit data, and four channels of data are added to produce 14-bit data. When the calculation data 103 to be output to the calculation section 12 is expressed in bit rate, (1) when data is not reduced, the bit rate is as shown in the following 401; (2) when two channels of data are combined, the bit rate is as shown in the following 402; and (3) when four channels of data are combined, the bit rate is as shown in the following 403.

$$401:\ 64\mathrm{chs} \times 12\ \mathrm{bits} \times 50\ \mathrm{MHz} = 38.4\ \mathrm{Gbps}$$

$$402:\ 32\mathrm{chs} \times 13\ \mathrm{bits} \times 50\ \mathrm{MHz} = 20.8\ \mathrm{Gbps}$$

$$403:\ 16\mathrm{chs} \times 14\ \mathrm{bits} \times 50\ \mathrm{MHz} = 11.2\ \mathrm{Gbps}$$

The more the number of channels is reduced, proportionally, the more the transfer rate of the calculation data 103 is reduced. Note that addition and combining of adjacent channels of echo detection data 101 can reduce the transfer rate of the calculation data 103, but inevitably involve degradation of image quality. In other words, the transfer rate and the image quality of the calculation data 103 is a tradeoff. In light of this, the present embodiment is configured such that a plurality of reduction processes each having a different data reduction amount can be performed at the same time, and a selector 37 which is a switching unit is provided so that an operator can arbitrarily set a data transfer rate (image quality). More specifically, the present embodiment is configured such that while actually viewing an ultrasound image displayed on the image display section 9, the operator can arbitrarily switch the number of additions of adjacent channels using the selector switching output 106 of the transmitting/receiving control section 2 from the input operation section 1.

As described above, the data reducing section 11 which adds the adjacent channels can be made to change the transfer rate (data reduction amount) of the calculation data 103 to the calculation section 12 and thereby the operator can arbitrarily reduce the data transfer time and the calculation time. Note that the above description has provided two choices (three choices including no reduction) of reduction processes: one for reducing two channels and the other for reducing four channels, but three or more reduction processes may be selectable. Note that the number of additions of channels is not necessarily power of 2, but adjacent three or five channels of echo detection data may be added and combined.

(Second embodiment)

The first embodiment focuses on the reduction process by addition of adjacent channels, but the second embodiment will focus on the reduction process by controlling a sampling frequency as follows. Note that the configuration other than the data reducing section 11 is the same as the configuration of the first embodiment, and thus the description thereof will be omitted.

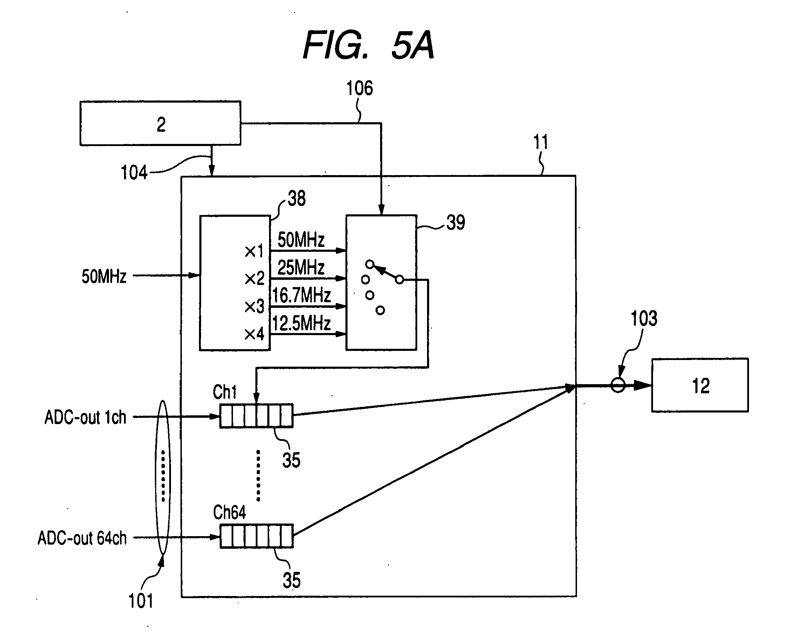

FIG. 5A illustrates a configuration of the data reducing section 11 by a sampling frequency. The echo detection data 101 sampled by the A/D converter 34 of the receiving section 4 is written to an FIFO 35 of the data reducing section 11 in synchronism with a 50 MHz sampling clock frequency. The data reducing section 11 includes an input clock frequency divider 38 and a selector 39 capable of selecting a sampling clock frequency. The data reducing section 11 can perform time interval reduction process using a clock frequency selected by the selector 39 from the echo detection data 101 written to the FIFO 35 to output the reduced calculation data 103 to the calculation section 12. Note that the selector 39 can be selected based on a selector switching output 106 from the transmitting/receiving control section 2. Like the first embodiment, in the present embodiment, the operator can also arbitrarily switch the selector 39 by operating the input operation section 1.

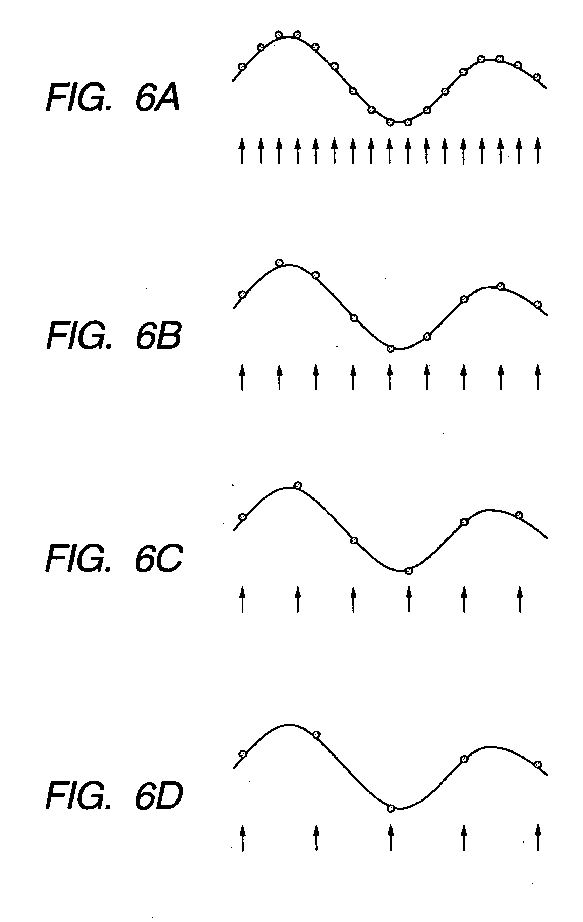

FIGS. 6A, 6B, 6C, and 6D each is a plot chart when the echo detection data 101 is input at a sampling frequency of 50 MHz, 25 MHz, 16.7 MHz, and 12.5 MHz respectively. The respective bit rates of the calculation data 103 are as follows.
FIG. 6A 50 MHz: 12 bits × 64 chs × 50 MHz = 38.4 Gbps
FIG. 6B 25 MHz: 12 bits × 64 chs × 25 MHz = 19.2 Gbps
FIG. 6C 16.7 MHz: 12 bits × 64 chs × 16.7 MHz = 12.8 Gbps
FIG. 6D 12.5 MHz: 12 bits × 64 chs × 12.5 MHz = 9.6 Gbps

When the sampling frequency is 50 MHz, no reduction process is performed and thus the bit rate becomes maximum. When the sampling frequency is 25 MHz, 16.7 MHz, and 12.5 MHz, the bit rate thereof becomes 1/2, 1/3, and 1/4 of the maximum respectively.

Note that reduction in sampling clock frequency can reduce the transfer rate of the calculation data 103, but increases the time interval and thus inevitably involves degradation of image quality of an ultrasound image. In other words, the transfer rate of the calculation data 103 and the image quality is a tradeoff. According to the present embodiment, the operator can arbitrarily set the data reduction amount by switching the selector 39, which is a switching unit, and thus the operator can obtain an ultrasound image of a desired image quality. More specifically, when the operator operates the input operation section 1 while actually viewing an ultrasound image displayed on the image display section 9, the selector switching output 106 corresponding to this operation is output to the selector 39 from the transmitting/receiving control section 2, and thus the image quality (data transfer rate) can be arbitrarily switched.

Alternatively, the calculation section 12 may generate original sampling rate data by interpolating the calculation data 103 received from the data reducing section 11. This is effective when calculation by the calculation section 12 such as partial correlation matrix calculations can be performed at high speeds but data transfer is a bottleneck. Such interpolation can produce high time resolution images. In addition, interpolation can be switched on or off according to the operation of the operator.

As described above, the data reducing section 11 which switches sampling clock frequencies can be made to change the transfer rate of the calculation data 103 to the calculation section 12 and thereby the operator can arbitrarily reduce the data transfer time and the calculation time. Note that in the above description, three kinds of reduction processes (25 MHz, 16.7 MHz, and 12.5 MHz) are selectable, but four or more reduction processes may be selectable.

Note also that the sampling frequency is not necessarily one over an integer of a sampling frequency (here 50 MHz) of the echo detection data 101, but may be one over a non-integer (e.g., 1/2.5, namely, 20 MHz) using a non-integral frequency divider. More specifically, the A/D converter 34 of the receiving section 4 is made to variably control a sampling frequency when an analog signal 100 is converted to a digital signal and thereby the same or higher function as the reduction process by a digital signal of the data reducing section 11 as described in FIG. 5A can be provided.

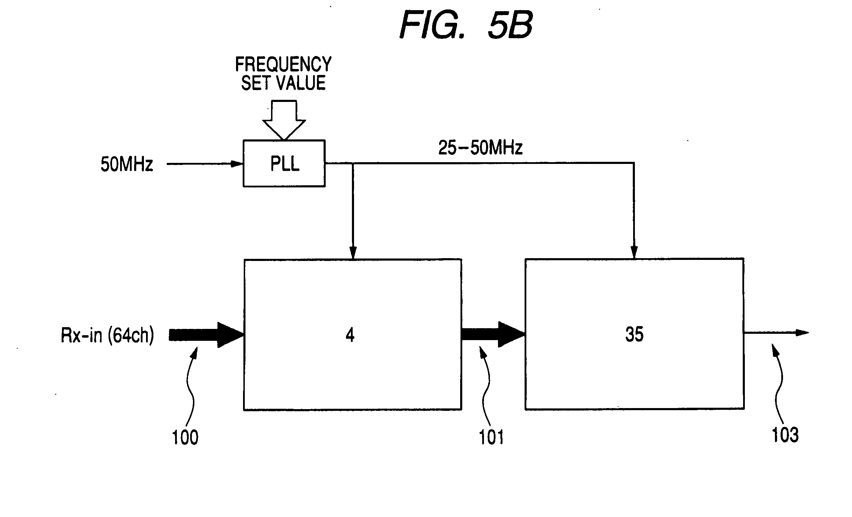

FIG. 5B illustrates a method of variably controlling a sampling frequency when the analog signal 100 is converted to a digital signal 101. In this case, unlike the reduction process by a digital signal, a PLL (Phase Locked Loop) clock generator is used as a clock generator (generator which generates an instruction signal instructing a sampling frequency to the receiving section) generating a sampling frequency. The PLL clock generator allows an oscillation frequency to be freely set and thus the sampling frequency can be set in smaller units.

The PLL clock generator is a device which can oscillate a signal having an accurately synchronized frequency by detecting a phase difference between an input signal serving as a reference frequency (50 MHz) and an output signal and controlling a VCO (oscillator changing a frequency by a voltage) and the loop of a circuit. By supplying a frequency setting value to this device, the sampling frequency can be set in several Hz units within a range of 25 to 50 MHz. Thus, the bit rate of the calculation data 103 can be dynamically variably controlled.

That is, the PLL clock generator can change the sampling frequency to a frequency lower than the reference sampling frequency. Thus, a change to the lower sampling frequency reduces the amount of data transferred to the calculating unit. In other words, in the configuration of FIG. 5B, the receiving section itself functions as the data reducing unit. Moreover, the operator can arbitrarily set the sampling frequency using a dial of the input operation section 1 and thus can change the bit rate more flexibly than using the selector 39.

(Modifications)

The data reduction process is not limited to the aforementioned methods of the first and second embodiments, but data reduction process may be performed by truncating the lower order bits (reducing the number of bits) of the echo detection data 101. For example, when 12-bit echo detection data 101 is reduced to 11-bit data, the bit rate is as follows.

```
11 bits × 64chs × 50 MHz = 35.2 Gbps
```

Alternatively, a method is sufficiently effective in which the data reducing section 11 performs a data compression process and the calculation section 12 performs a data expansion process. In addition, a method is also effective in which any two or more methods are combined from among the method of combining data between adjacent channels (first embodiment), the method of reducing a sampling frequency (second embodiment), the method of truncating the lower order bits, and the method of compressing data.

(Third embodiment)

The first and second embodiments of the present invention describe the imaging using the DCMP method and the spatial smoothing, but the operator may prefer images by the phase matching calculation by the conventional ultrasound diagnostic apparatuses (FIGS. 2 and 3) without using the adaptive signal processing. That is, it can be expected that there are some operators who think it easier to detect an abnormal organ by a familiar image quality not by a high resolution image quality. It is often after comparing actual images when it is determined which imaging method is better for detecting. Thus, the ultrasound diagnostic apparatus according to the present embodiment displays images using two or more imaging methods on the screen.

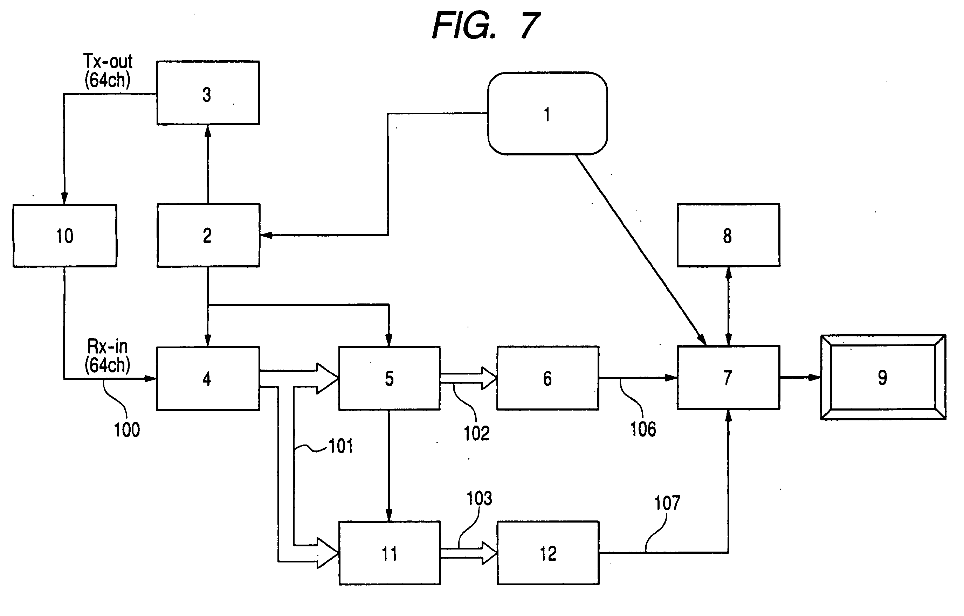

FIG. 7 is a block diagram illustrating a configuration of an ultrasound diagnostic apparatus according to the present embodiment. The ultrasound diagnostic apparatus of the present embodiment is configured as an apparatus having both functions of, the phase matching calculation section 5 and the signal processing section 6 same as the conventional configuration, and the data reducing section 11 and the calculation section 12 same as the configurations of the first and the second embodiments.

The echo detection data 101 outputs from the receiving section 4 is sent to both the phase matching calculation section 5 and the data reducing section 11. Both processes are performed in parallel and two kinds of image data 106 and 107 are sent to the scan converter 7. The scan converter 7 temporarily stores the two kinds of image data 106 and 107 in the image data storage section 8. Then, the scan converter 7 selects the stored two kinds of image data to display two images at the same time or one by one on the image display section 9.

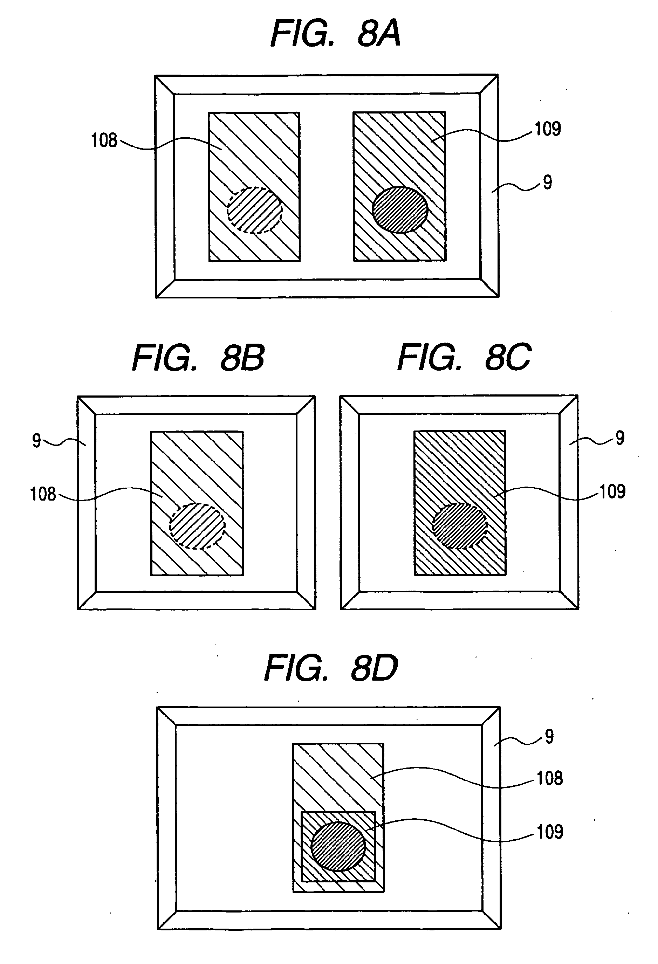

FIG. 8A illustrates an image display method (1) in which two kinds of image data are output side by side to the image display section 9. Both images are simultaneously displayed on one screen on which an image 108 subjected to the conventional phase matching is displayed on the left side and an image 109 subjected to the adaptive signal processing is displayed on the right side. Even the same organ to be observed appears differently due to the difference in image processing. Therefore, when two images are displayed at the same time, it is easier to compare the two images and thereby diagnostic image accuracy can be improved.

FIGS. 8B and 8C each illustrates an image display method (2) in which the two images are switched to be output to the image display section 9 in FIGS. 8B and 8C, respectively. The image 108 subjected to the conventional phase matching illustrated in FIG. 8B and the image 109 subjected to the adaptive signal processing illustrated in FIG. 8C may be alternately switched to be displayed on one screen. Even the same organ to be observed appears differently due to the difference in image processing. Therefore, when two images are switched to be displayed on the same screen, it is easier to compare the two images and thereby diagnostic image accuracy can be improved.

FIG. 8D illustrates an image display method (3) in which two images overlappedly output to the image display section 9. The image 109 in which the necessary region of interest has been subjected to the adaptive signal processing is overlapped with the image 108 subjected to the conventional phase matching to be displayed on one screen. Even the same organ to be observed appears differently due to the difference in image processing. Therefore, when only the necessary region of interest is overlappedly displayed, it is easier to compare the two images and thereby diagnostic image accuracy can be improved.

Note that the image display methods (1) to (3) focus on the two-screen display of the conventional image 108 and the image 109 subjected to the adaptive signal processing, but the image display methods can also be applied likewise to displaying two or more images each having a different reduction amount (i.e., image quality). Moreover, when images are generated by temporally switching between the channel sum reduction of the first embodiment and the sampling reduction of the second embodiment (for example, every screen), a plurality of images each generated by a different method can be simultaneously output to the image display section 9 in one screen.

Moreover, for example, if it is not a problem that only the region of interest needs to be displayed at high resolution but the other regions are left displayed at low resolution, adaptive processing can be advantageously performed only on the necessary portions by performing speed focus processing without lowering the entire frame rate.

Moreover, a method is effective in which, when the ultrasound probe is operated with respect to the object, the motion thereof is detected using a motion sensor and the like; and the reduction process is switched between a high resolution display at low-speed movement and a speed focus display at high-speed movement.

Further, the ultrasound probe itself may include a selector switch, in addition to a keyboard and a pointing device of the input operation section 1 to which the selector switch is generally included, in order to switch the image display methods. With this configuration that the ultrasound probe itself includes an operation section, the operator can switch the screen while operating the ultrasound probe with respect to the object. Thus this is effective in improving operating efficiency.

As described above, the scan converter 7 displaying images on the screen is made to switch the display methods to the image display section 9, and thereby the operator can observe a plurality of diagnostic images at the same time while comparing the diagnostic images. Note that the switching unit of the present invention can also be configured such that when the adaptive signal processing is performed on a received signal, switching can be made between the operation with the reduction process of the present invention and the operation without the reduction process of the present invention.

Moreover, the "adaptive signal processing" of the present invention is not limited to the DCMP method and the spatial smoothing used in the embodiments. Any well known general adaptive signal processing in the field of radar causes a problem with increased data amount when applied to a measurement apparatus, and thus is within the scope of the present invention.

Moreover, "ultrasound" of the present invention is a concept including not only an echo ultrasound reflected inside an object when an ultrasound is emitted from an ultrasound probe to the object, but also a photoacoustic wave which is a elastic wave generated by an expansion of a light absorbing body inside an object by pulsed light emitted to the object.

The present invention is not limited to the above embodiments and various changes and modifications can be made within the scope of the present invention. Therefore to apprise the public of the scope of the present invention, the following claims are made.

**Claims**

1. A measurement apparatus generating image data inside an object using an analog signal obtained by receiving an ultrasound propagated inside the object with a plurality of ultrasound transducing devices, the measurement apparatus comprising:

   a received signal processing unit (4) which converts the analog signal to a digital signal;
   a calculating unit (12) which performs adaptive signal processing using spatial smoothing on the digital signal and generates image information; and
   a data reducing unit (11) which reduces data amount of the digital signal transferred from the received signal processing unit to the calculating unit, **characterized in that**
   the data reducing unit (11) executes a plurality of data reduction processes by temporally switching among the data reduction processes, and
   the measurement apparatus further comprises a switching unit (37) used for displaying, simultaneously or in an alternating succession, images generated based on signals on which different data reduction processes are performed.

2. The measurement apparatus according to claim 1, wherein
   the data reducing unit (11) can execute a plurality of data reduction processes each having a different data reduction amount, and
   the measurement apparatus further comprises a selecting unit (39) capable of selecting a data reduction process performed by the data reducing unit.

3. The measurement apparatus according to any one of claims 1 and 2, wherein the data reducing unit (11) reduces data amount by adding received signals between adjacent ultrasound transducing devices.

4. The measurement apparatus according to any one of claims 1 to 3, wherein the data reducing unit (11) reduces data amount by reducing a sampling frequency of a digitally converted received signal.

5. The measurement apparatus according to claim 4, wherein the calculating unit (12) interpolates reduced data.

6. A measurement apparatus according to claim 4, further comprising:

   a generator (2) which generates an instruction signal instructing a sampling frequency to the received signal processing unit (4) when the conversion from an analog signal to a digital signal is performed,
   wherein the generator can change the sampling frequency to a lower sampling frequency than a reference sampling frequency and reduces an amount of data transferred to the calculating unit (12) by changing to the lower sampling frequency.

7. The measurement apparatus according to any one of claims 1 to 6, further comprising:

   a delay and sum unit (5) which matches a phase of the signal to be received;
   a signal processing section (6) which performs signal processing on the signal that is phase-matched and generates an image; and
   a switching unit (7) used for displaying an image generated by the calculating unit and an image generated by the signal processing section, simultaneously or in an alternating succession.

**Patentansprüche**

1. Messvorrichtung, die Bilddaten innerhalb eines Objekts erzeugt, unter Verwendung eines analogen Signals, das durch Empfangen von Ultraschall, der sich innerhalb des Objekts ausgebreitet hat, mit einer Vielzahl von Ultraschallempfangseinrichtungen erhalten wird, wobei die Messvorrichtung aufweist:

   eine Einheit zum Verarbeiten eines empfangenen Signals (4), die das analoge Signal in ein digitales Signal umwandelt;
   eine Berechnungseinheit (12), die eine adaptive Signalverarbeitung unter Verwendung von räumlicher Glättung bezüglich des digitalen Signals durchführt und Bildinformationen erzeugt;
   einer Datenreduzierungseinheit (11), die eine Datenmenge des digitalen Signals, das von der Einheit zum Verarbeiten eines empfangenen Signals an die Berechnungseinheit übertragen wird, reduziert, **dadurch gekennzeichnet, dass**
   die Datenreduzierungseinheit (11) eine Vielzahl von Datenreduzierungsprozessen durch vorübergehendes Umschalten unter den Datenreduzierungsprozessen durchführt, und
   die Messvorrichtung weiterhin eine Schalteinheit (37) aufweist, die zum Anzeigen, gleichzeitig oder in wechselnder Abfolge, von Bildern verwendet wird, die basierend auf Signalen erzeugt werden, bezüglich denen verschiedene Datenreduzierungsprozesse durchgeführt werden.

2. Messvorrichtung gemäß Anspruch 1, wobei
   die Datenreduzierungseinheit (11) eine Vielzahl von Datenreduzierungsprozessen ausführen kann, die jeweils einen unterschiedlichen Datenreduzierungsbetrag aufweisen, und
   die Messvorrichtung weiterhin eine Auswahleinheit (39) aufweist, die dazu in der Lage ist, einen Datenreduzierungsprozess, der durch die Datenreduzierungseinheit durchgeführt wird, auszuwählen.

3. Messvorrichtung gemäß einem der Ansprüche 1 und 2, wobei die Datenreduzierungseinheit (11) eine Datenmenge durch Addieren von empfangenen Signalen zwischen benachbarten Ultraschallempfangseinrichtungen reduziert.

4. Messvorrichtung gemäß einem der Ansprüche 1 bis 3, wobei die Datenreduzierungseinheit (11) eine Datenmenge durch Reduzieren einer Abtastfrequenz eines digital gewandelten empfangenen Signals reduziert.

5. Messvorrichtung gemäß Anspruch 4, wobei die Berechnungseinheit (12) reduzierte Daten interpoliert.

6. Messvorrichtung gemäß Anspruch 4, weiterhin mit:

   einem Generator (2), der ein Anweisungssignal erzeugt, das der Einheit zum Verarbeiten eines empfangenen Signals (4) eine Abtastfrequenz anweist, wenn die Umwandlung von einem analogen Signal in ein digitales Signal durchgeführt wird,
   wobei der Generator die Abtastfrequenz in eine niedrigere Abtastfrequenz als eine Referenzabtastfrequenz ändern kann und eine Menge an Daten, die an die Berechnungseinheit übertragen wird, durch Ändern in die niedrigere Abtastfrequenz reduziert.

7. Messvorrichtung gemäß einem der Ansprüche 1 bis 6, weiterhin mit:

   einer Verzögerungs- und Summierungseinheit (5), die eine Phase des zu empfangenen Signals anpasst;
   einem Signalverarbeitungsabschnitt (6), der eine Signalverarbeitung bezüglich des Signals, das phasen-angepasst ist, durchführt, und ein Bild erzeugt; und
   einer Schalteinheit (7), die zum Anzeigen eines Bildes, das durch die Berechnungseinheit erzeugt wird, und eines Bildes, das durch den Signalverarbeitungsabschnitt erzeugt wird, gleichzeitig oder in wechselnder Abfolge, verwendet wird.

**Revendications**

1. Appareil de mesure générant des données d'image à l'intérieur d'un objet en utilisant un signal analogique obtenu par réception d'un ultrason propagé à l'intérieur de l'objet avec une pluralité de dispositifs de transduction d'ultrason, l'appareil de mesure comprenant :

   une unité (4) de traitement de signal reçu qui convertit le signal analogique en un signal numérique ;
   une unité (12) de calcul qui effectue un traitement adaptatif de signal en utilisant un lissage spatial sur le signal numérique et qui génère de l'information d'image ; et
   une unité (11) de réduction de données qui réduit la quantité de données du signal numérique transféré de l'unité de traitement de signal reçu à l'unité de calcul, caractérisé en ce qui :

   l'unité (11) de réduction de données exécute une pluralité de traitements de réduction de données en commutant temporairement entre les traitements de réduction de données ; et
   l'appareil de mesure comprenant en outre une unité (37) de commutation utilisée pour afficher, simultanément ou en succession alternée, des images générées basées sur des signaux sur lesquels sont effectués des traitements différents de réduction de données.

2. Appareil de mesure selon la revendication 1, dans lequel :

   l'unité (11) de réduction de données peut exécuter une pluralité de traitements de réduction de données ayant chacun une quantité différente de réduction de données, et
   l'appareil de mesure comprenant en outre une unité (39) de sélection capable de sélectionner un traitement de réduction de données effectué par l'unité de réduction de données.

3. Appareil de mesure selon l'une quelconque des revendications 1 et 2, dans lequel l'unité (11) de réduction de données réduit la quantité de données en additionnant des signaux reçus entre des dispositifs adjacents de transduction d'ultrason.

4. Appareil de mesure selon l'une quelconque des revendications 1 à 3, dans lequel l'unité (11) de réduction de données réduit la quantité de données en réduisant la fréquence d'échantillonnage d'un signal reçu converti en numérique.

5. Appareil de mesure selon la revendication 4, dans lequel l'unité (12) de calcul interpole les données réduites.

6. Appareil de mesure selon la revendication 4, comprenant en outre :

   un générateur (2) qui génère un signal d'instruction ordonnant une fréquence d'échantillonnage à l'unité (4) de traitement de signal reçu lorsque se fait la conversion d'un signal analogique en un signal numérique,
   dans lequel le générateur peut changer la fréquence d'échantillonnage pour une fréquence d'échantillonnage plus basse qu'une fréquence d'échantillonnage de référence et réduit la quantité de données transférées à l'unité (12) de calcul en changeant à la fréquence d'échantillonnage plus basse.

7. Appareil de mesure selon l'une quelconque des revendications 1 à 6, comprenant en outre :

   une unité (5) de retardement et de totalisation qui adapte la phase du signal à recevoir ;
   une section (6) de traitement de signal qui effectue un traitement de signal sur le signal qui est adapté en phase et génère une image ; et
   une unité (7) de commutation utilisée pour afficher une image générée par l'unité de calcul et une image générée par la section de traitement de signal, simultanément ou en succession alternée.

## FIG. 1

EP 2 435 850 B1

FIG. 2

EP 2 435 850 B1

# FIG. 3

FIG. 4

FIG. 5A

EP 2 435 850 B1

FIG. 5B

FIG. 6A

FIG. 6B

FIG. 6C

FIG. 6D

# FIG. 7

EP 2 435 850 B1

## FIG. 8A

108 — 109

9

## FIG. 8B

9

108

## FIG. 8C

9

109

## FIG. 8D

9

108

109

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6798380 B
- US 5349525 A

- US 5970025 A

**Non-patent literature cited in the description**

- *IEEE Trans. Acoust., Speech, Signal Process,* June 1985, vol. 33 (3), 527-536